# EUROPEAN PATENT SPECIFICATION

(11) **EP 3 236 859 B1**
(45) Date of publication and mention of the grant of the patent: **31.03.2021**
(21) Application number: 15817563.8
(22) Date of filing: 10.12.2015
(51) Int. Cl.: A61B 8/08, A61B 8/00

(54) **NEEDLE TRAJECTORY PREDICTION FOR TARGET BIOPSY**
NADELWEGVORHERSAGE ZUR ZIELBIOPSIE
PRÉDICTION DE TRAJECTOIRE D'AIGUILLE POUR BIOPSIE CIBLE

(30) Priority: 24.12.2014 US 201462096569 P
(43) Date of publication of application: 01.11.2017
(73) Proprietor: Koninklijke Philips N.V., 5656 AG Eindhoven (NL)
(72) Inventor: LU, Huanxiang, 5656 AE Eindhoven (NL); LI, Junbo, 5656 AE Eindhoven (NL); VIGNON, Francois Guy Gerard Marie, 5656 AE Eindhoven (NL); JAIN, Ameet Kumar, 5656 AE Eindhoven (NL); WU, Ying, 5656 AE Eindhoven (NL)
(74) Representative: Philips Intellectual Property & Standards
(86) International application number: PCT/IB2015/059494
(87) International publication number: WO 2016/103094

(56) References cited:
- WO-A1-97/03609
- WO-A1-2005/055849
- WO-A1-2011/138698
- WO-A1-2012/172458
- WO-A2-2006/116163
- JP-A- H1 176 241
- US-A1- 2005 159 676

## Description

The present invention generally relates to ultrasound-guided target biopsies (e.g., liver biopsy, renal biopsy, etc.). The present invention specifically relates to a prediction of a needle trajectory during a target biopsy procedure.

Ultrasound guidance is widely used for target biopsies to increase the accuracy of the procedure and reduce the potential risk of medical accidents. In such procedures, a needle is inserted into the patient aiming at the biopsy target. In the meanwhile, clinicians usually need to estimate the triggered needle trajectory before firing the biopsy gun in order to know if the needle will puncture through the target tissue. The trajectory is approximately an extension of certain centimeters along the needle shaft estimated from the prior knowledge of the needle parameter. Thus, when the needle is clearly visible in the ultrasound image, it may be relatively easy for the physicians to estimate the trajectory. However, in deep organs (e.g., liver and kidney), needles are usually invisible in the ultrasound image due to their specular nature and unfavorable incidence angles, which results in difficulties in estimating the needle trajectory. Moreover, the needle is not always in the ultrasound image plane during the procedure due to the hand motion of the clinician and breathing motion of the patient, which provides more difficulties in estimating the needle trajectory .WO2012/172458A1 discloses a system for imaging medical device comprising a trilateration module that interprets signals sensed between tracking element and array of transducers to compute times of flight of signals associated with array of transducers. US 2005/159676 A1 discloses a targeted biopsy system which allows planning of tissue to be sampled, targeting of specific areas of tissue in reference to the plan, capturing the tissue sample and recording the source location of the tissue sample, particularly for use in collecting tissue samples from the prostate gland.

To enhance the visualization of interventional tools in ultrasound image, an ultrasound-based tracking technology has been proposed to track a tip of an interventional tool by embedding small ultrasound receivers near the tip of the interventional tool. The position of the interventional tool is then estimated by processing the signal received by these ultrasound receivers, which is then visualized on the ultrasound image. The present invention enhances such ultrasound-based tracking technology by providing a precise prediction of a three-dimensional ("3D") in-plane biopsy trajectory or a 3D out-of-plane biopsy trajectory on the ultrasound image.

One form of the present invention is a target biopsy system according to claim 4. In operation, the ultrasound probe projects an ultrasound plane intersecting an anatomical region (e.g. an abdominal region, a cranial region, a mammary region, an abdominal region, etc.). The ultrasound receiver(s) sense the ultrasound plane as the target biopsy needle is inserted into the anatomical region. In response to the ultrasound receiver(s) sensing the ultrasound plane, the ultrasound guide controller predicts a biopsy trajectory of the target biopsy needle within the anatomical region relative to ultrasound plane. The prediction indicates the biopsy trajectory is either within the ultrasound plane (i.e., an in-plane biopsy trajectory) or outside of the ultrasound plane (i.e., an out-of-plane biopsy trajectory).

For purposes of the present invention, the term "ultrasound probe" broadly encompasses any ultrasound probe as known in the art employing one or more ultrasound transducers/transmitters/receivers for projecting an ultrasound plane intersecting the anatomical region. Examples of an ultrasound probe include, but are not limited to, two-dimensional and three-dimensional ultrasound probes with sector, curvilinear or linear geometries.

For purposes of the present invention, the term "target biopsy needle" broadly encompasses any type of biopsy needle as known in the art employing a stylet or the like to thereby cut a tissue sample when the target biopsy needle is inserted into the anatomical region. Examples of a target biopsy needle include, but is not limited to, guillotine-type biopsy needles with a firing or "gun" mechanism used for core biopsy (e.g., a Bio-Cut® or Bard Magnum® biopsy needle).

For purposes of the present invention, terms of the art including, but not limited to, "in-plane", "out-of-plane", "receiver", and "biopsy trajectory" are to be interpreted as known in the art of the present invention and exemplary described herein. More particularly, the term "receiver" is inclusive of a receiver and a transceiver as known in the art.

For purposes of the present invention, the term "ultrasound guide controller" broadly encompasses all structural configurations of an application specific main board or an application specific integrated circuit housed within or linked to a computer or another instruction execution device/system for controlling an application of various inventive principles of the present invention as subsequently described herein. The structural configuration of the ultrasound guide controller may include, but is not limited to, processor(s), computer-usable/computer readable storage medium(s), an operating system, peripheral device controller(s), slot(s) and port(s). Examples of a computer includes, but is not limited to, a server computer, a client computer, a workstation and a tablet.

A second form of the present invention is the ultrasound guide controller according to claim 1. In operation, the ultrasound probe generates an ultrasound image of an anatomical region responsive to ultrasound data from the ultrasound probe representative of the ultrasound plane intersecting an anatomical region. The receiver tracking module tracks a position of each ultrasound receiver relative to the ultrasound image of the anatomical region responsive to sensing data from the ultrasound receivers representative of a sensing of the ultrasound plane as the target biopsy needle is inserted into the anatomical region. The needle trajectory module predicts the biopsy trajectory of the target biopsy needle relative to the ultrasound plane responsive to the tracked positions of the ultrasound receivers relative to the ultrasound image of the anatomical region.

For purposes of the present invention, the term "module" broadly encompasses an application component of the ultrasound guide controller consisting of an electronic circuit or an executable program (e.g., executable software and/firmware).

Another example is a target biopsy method involving (1) the ultrasound probe projecting the ultrasound plane intersecting the anatomical region, (2) the ultrasound receivers sensing the ultrasound plane as a target biopsy needle is inserted into the anatomical region, and (3) the ultrasound guide workstation predicting a biopsy trajectory of the target biopsy needle within the anatomical region relative to the ultrasound plane.

The foregoing forms and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims.
FIG. 1 illustrates an exemplary embodiment of a target biopsy system in accordance with the present invention.
FIGS. 2-4 illustrate exemplary visualizations of a predicted needle trajectory by the target biopsy system of FIG. 1.

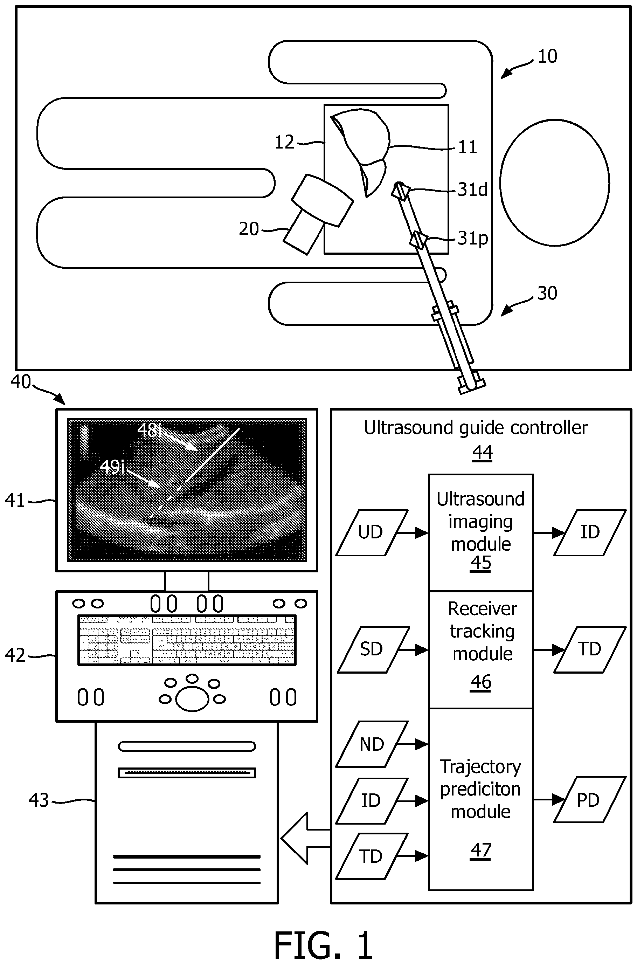

To facilitate an understanding of the present invention, exemplary embodiments of the target biopsy system of the present invention will be provided herein for an ultrasound guided target biopsy procedure for a liver 11 of a patient 10 as shown in FIG. 1. From the description of the exemplary embodiments of the present invention, those having ordinary skill in the art will appreciate how to make and use the target biopsy system of the present invention for any type of ultrasound-guided target biopsy procedure (e.g., prostate, kidney, breast etc.) involving various types of ultrasound probes and target biopsy needles.

For purposes of the present invention, terms of the art including, but not limited to, "firing mechanism", "co-axial introducer" and "tracked position" are to be interpreted as known in the art of the present invention and exemplary described herein.

Referring to FIG. 1, the ultrasound-guided target biopsy, which does not form part of the present invention, involves an ultrasound probe 20 and a target biopsy needle 30 for extracting tissue from liver 11 of patient 10 as known in the art.

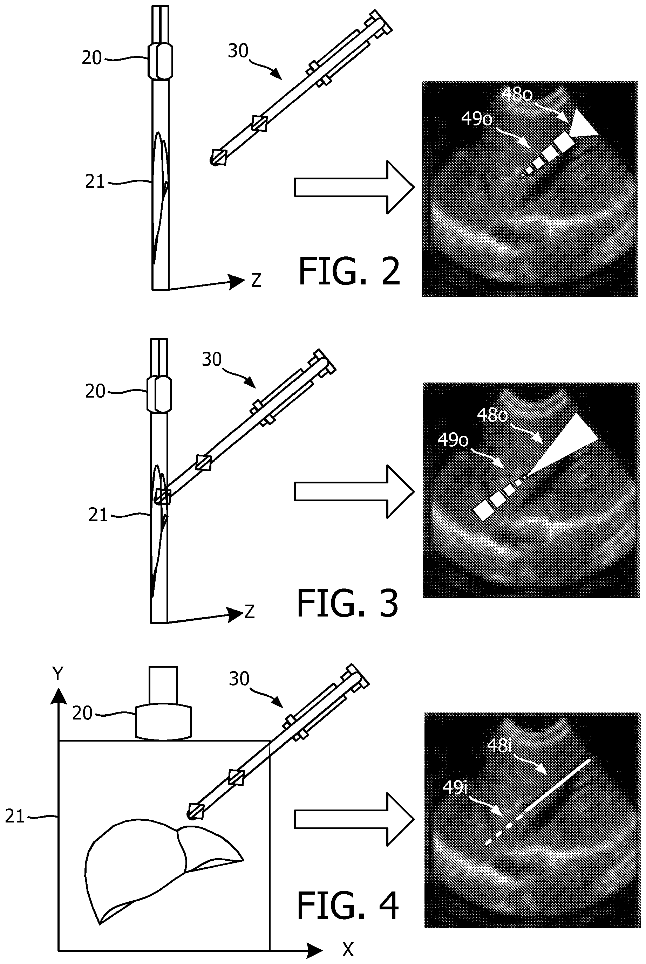

Ultrasound probe 20 employs one or more ultrasound transducers, transmitters receivers and/or transceivers for projecting an ultrasound plane intersecting an abdominal region 12 (e.g., ultrasound plane 21 as shown in FIG. 2). Examples of ultrasound probe 20 include, but are not limited to, two-dimensional and three-dimensional ultrasound probes with sector, curvilinear or linear geometries.

Target biopsy needle 30 employs a stylet or the like to thereby cut a tissue sample of liver 11 when needle 30 is inserted into abdominal region 12. Examples of target biopsy needle 30 include, but is not limited to, guillotine-type biopsy needles with an automatic/semi-automatic firing or "gun" mechanism used for core biopsy (e.g., a Bio-Cut® or Bard Magnum® biopsy needle). When included, a fire mechanism is operated to project target biopsy needle along a biopsy trajectory of target biopsy needle within abdominal region 12.

Two or more ultrasound receivers 31 (i.e., a receiver or a transceiver) for sensing the ultrasound plane are attached as target biopsy needle 30 is being inserted within abdominal region 12 of patient 10. As known in the art, a degree of sensing the ultrasound plane is a function of a distance between an ultrasound receiver 31 and the ultrasound plane.

In practice, ultrasound receivers 31 are spatially arranged on biopsy needle 30 suitable for facilitating a distinctive sensing of the ultrasound plane by each ultrasound receiver 31. In one embodiment, as shown in FIG. 1, distal ultrasound receiver 31d is attached to/embedded within target biopsy needle 30 adjacent a tip of target biopsy needle 30 and a proximal ultrasound receiver 31p is attached to/embedded within target biopsy needle 30 is a middle of shaft of target biopsy needle 30. In an alternative embodiment, target biopsy needle 30 includes a coaxial introducer through which target biopsy needle 30 into abdominal region 12 with receivers 31 being attached to/embedded within the coaxial introducer.

The ultrasound-guided target biopsy procedure involves an ultrasound guide machine 40 employing a monitor 41, an interface platform 42, a workstation 43 and a ultrasound guide controller 44 installed within workstation 43. While not shown, in practice, ultrasound probe 20 and ultrasound receivers 31 are connected/coupled to workstation 43 in any manner as known in the art.

Ultrasound guide controller 44 includes and/or is accessible by an operating system (not shown) as known in the art for controlling various graphical user interfaces, data and images on monitor 41 as directed by a workstation operator (e.g., a doctor, technician, etc.) via a keyboard, buttons, dials, joysticks, etc. of interface platform 42, and for storing/reading data as programmed and/or directed by the workstation operator of interface platform 42.

Ultrasound guide controller 44 further executes application modules including an ultrasound imaging module 45, a receiver tracking module 46, and a trajectory prediction module 47 for implementing an ultrasound guided target biopsy procedure of liver 11.

Specifically, ultrasound imaging module 45 is structurally configured to receive ultrasound data *UD* from ultrasound probe 20 representative of the ultrasound plane intersecting abdominal region 12 of patient 11, and to execute a known process for generating a planar ultrasound image of abdominal region 12 for display by monitor 41 as shown.

Receiver tracking module 46 is structurally configured to sense data *SD* from ultrasound receivers 31 representative of a sensing of the ultrasound plane as the target biopsy needle 30 is inserted into abdominal region 12 of patient 11, and to execute a known process for tracking a position of each ultrasound receiver 31 relative to the ultrasound plane intersecting abdominal region 12. For each ultrasound receiver 31, the tracked position indicates whether the particular ultrasound receiver 31 is within the ultrasound plane (i.e., in-plane) or outside of the ultrasound plane (i.e., out-of-plane). More particularly, the sensing of the ultrasound plane of the particular ultrasound receiver 31 will indicate a three-dimensional ("3D") position of each ultrasound receiver 31 in terms of height, width and depth whereby in-plane has zero (0) depth and out-of-plane has a non-zero depth.

Trajectory prediction module 47 is structurally configured to receive needle data *ND,* pre-operatively or intra-operatively, representative of a dimension/configuration profile of target biopsy needle 30 whereby parameters of needle 30 are known for determining an orientation of needle 30 relative to the ultrasound plane intersecting abdominal region 12 including, but not limited to, (1) a length of needle 30 prior to and subsequent to a firing of needle 30 and (2) an attachment point of each ultrasound receiver 31.

Trajectory prediction module 47 is further structurally configured to receive image data *ID* from ultrasound imaging module 45 representative of the planar ultrasound image of abdominal region 12 being displayed, and tracking data *TD* from receiver tracking module 46 representative of the tracked positions of ultrasound receivers 31 relative to the ultrasound plane intersecting abdominal region 12. In response thereto, trajectory prediction module 47 is further structurally configured to receive to predict a biopsy trajectory of target biopsy needle 30 relative to the ultrasound plane by executing a process of the present invention including:
(1) determining an orientation of a virtual version of an unfired needle 30 relative to the planar ultrasound image derived from a length of a virtual positioning of a segment of needle 30 between ultrasound receivers 31 relative to the planar ultrasound image as a function of the tracked positions of ultrasound receivers 31 ("orientation determination"); and
(2) determining a tip extension of a virtual version of a fired needle 30 previously oriented relative to the planar ultrasound image derived from a length of a virtual positioning of a fired stylet of needle 30 ("firing determination").

The orientation determination facilitates a generation by trajectory prediction module 47 of a needle overlay on the planar ultrasound image, and the firing determination facilitates a generation by trajectory prediction module 47 of a biopsy trajectory overlay on the planar ultrasound image. For example, as shown in FIG. 1, monitor 41 is displaying an in-plane needle overlay 48i and an in-plane biopsy trajectory overlay 49i when both ultrasound receivers 31 are in-plane of the ultrasound plane intersecting abdominal region 12.

In practice, the overlays may have any shape and/or any color indicative of an in-plane or out-of-plane sensing of needle 30 and the biopsy trajectory. For example, FIGS. 2-4 shows a sequence of needle 30 being transitioned from out-of-plane to in-plane relative to an ultrasound plane 21.

Specifically, FIG. 2 illustrates an initial insertion of needle 30 within abdominal region 12 (not shown) whereby both ultrasound receivers 31 are out-of-plane and an orientation of needle 30 is non-parallel to ultrasound plane 21. For this initial insertion, a needle overlay 48o of a white solid triangular shape based from a proximal end to distal end and a biopsy trajectory overlay 49o of a white dashed triangular shape based from an unfired needle tip to a fired needle tip illustrates both ultrasound receivers 31 are out-of-plane and that a fired needle tip would be out-of-plane.

FIG. 3 illustrates a further insertion of needle 30 within abdominal region 12 (not shown) whereby the distal ultrasound receiver 31 is in-plane, the proximal ultrasound receiver 31 is out-of-plane, and an orientation of needle 30 is non-parallel to ultrasound plane 21. For this further insertion, needle overlay 48o and biopsy trajectory overlay 49o illustrates the distal ultrasound receiver 31 is in-plane and the fired needle tip would be out-of-plane opposite needle 30.

FIG. 4 illustrates a rotation of ultrasound probe 20 relative to the insertion of needle 30 within abdominal region 12 (not shown) whereby both ultrasound receivers 31 are in-plane and therefore needle 30 in-plane to ultrasound plane 21. For this probe rotation, needle overlay 48i is a white line and biopsy trajectory overlay 49i is a dashed line illustrating the in-plane needle 30.

For the example of FIGS. 2-4, needle overlay 48o and biopsy trajectory overlay 49o may be colored red to indicate an out-of-plane needle 30, and needle overlay 48i is and biopsy trajectory overlay 49i may be colored green to indicate an in-plane needle 30.

Referring back to FIG. 1, trajectory prediction module 47 provides prediction data *PD* to the appropriate display module(s) of ultrasound guide controller 44 for display of the overlays on the ultrasound image. Additionally, prediction data *PD* may include a numerical readout of a distance of each ultrasound receiver 31 from the ultrasound plane and an angular orientation of needle 30 relative to the ultrasound plane to facilitate an operator of machine 40 in re-positioning ultrasound probe 20 and/or reinserting needle 30.

Referring to FIGS. 1-4, from the description of the exemplary embodiments of the present invention, those having ordinary skill in the art will appreciate numerous benefits of an intervention system of the present invention including, but not limited to, (1) application for various ultrasound-guided target biopsy procedures (e.g., liver biopsy, renal biopsy, etc.), particularly procedures whereby the biopsy needle is not clearly visible during the procedure, and (2) enhanced training for doctors in performing target biopsies.

Furthermore, as one having ordinary skill in the art will appreciate in view of the teachings provided herein, features, elements, components, etc. described in the present disclosure/specification and/or depicted in the FIGS. 1-4 may be implemented in various combinations of electronic components/circuitry, hardware, executable software and executable firmware, particularly as application modules of a controller as described herein, and provide functions which may be combined in a single element or multiple elements. For example, the functions of the various features, elements, components, etc. shown/illustrated/depicted in the FIGS. 1-4 can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, circuitry, combinations thereof, etc.) which is capable of (and/or configurable) to perform and/or control a process.

It will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Furthermore, exemplary embodiments of the present invention can take the form of a computer program product or application module accessible from a computer-usable and/or computer-readable storage medium providing program code and/or instructions for use by or in connection with, e.g., a computer or any instruction execution system. In accordance with the present disclosure, a computer-usable or computer readable storage medium can be any apparatus that can, e.g., include, store, communicate, propagate or transport the program for use by or in connection with the instruction execution system, apparatus or device. Such exemplary medium can be, e.g., an electronic, magnetic, optical, electromagnetic, infrared or semiconductor system (or apparatus or device) or a propagation medium. Examples of a computer-readable medium include, e.g., a semiconductor or solid state memory, magnetic tape, a removable computer diskette, a random access memory (RAM), a read-only memory (ROM), flash (drive), a rigid magnetic disk and an optical disk. Current examples of optical disks include compact disk - read only memory (CD-ROM), compact disk - read/write (CD-R/W) and DVD. Further, it should be understood that any new computer-readable medium which may hereafter be developed should also be considered as computer-readable medium as may be used or referred to in accordance with exemplary embodiments of the present invention and disclosure.

Having described preferred and exemplary embodiments of novel and inventive system for predicting a needle trajectory for target biopsy, (which embodiments are intended to be illustrative and not limiting), it is noted that modifications and variations can be made by persons having ordinary skill in the art in light of the teachings provided herein, including the FIGS 1-4. It is therefore to be understood that changes can be made in/to the preferred and exemplary embodiments of the present disclosure which are within the scope of the embodiments disclosed herein.

Moreover, it is contemplated that corresponding and/or related systems incorporating and/or implementing the device or such as may be used/implemented in a device in accordance with the present disclosure are also contemplated and considered to be within the scope of the present invention.

## Claims

1. A ultrasound guide controller (44) configured to control a target biopsy system that includes i) an ultrasound probe (20) operable to project an ultrasound plane intersecting an anatomical region and ii) a target biopsy needle (30) having at least two ultrasound receivers (31) arranged thereon in a known arrangement relative to the target biopsy needle (30) and in which each ultrasound receiver (31) is operable to sense the ultrasound plane as the target biopsy needle (30) is inserted into the anatomical region, the ultrasound guide controller (44) comprising:
an ultrasound imaging module (45), configured based on ultrasound data received from the ultrasound probe (20), to generate a planar ultrasound image of an anatomical region responsive to ultrasound data representative of an ultrasound plane intersecting the anatomical region;
a receiver tracking module (46), configured based on sense data received from the at least two ultrasound receivers (31), to track a position of each ultrasound receiver (31) relative to the ultrasound plane responsive to sensing data representative of a sensing of the ultrasound plane as the target biopsy needle (30) is inserted into the anatomical region; and
a trajectory prediction module (47) operable in communication with the ultrasound imaging module (45) and the receiver tracking module (46) to predict a biopsy trajectory of the target biopsy needle (30) relative to the ultrasound plane responsive to the tracked positions of the at least two ultrasound receivers (31) relative to the planar ultrasound image of the anatomical region; **characterized by** said prediction being based on
a) a known length of the target biopsy needle (30) prior to firing, b) a known length of the target biopsy needle (30) subsequent to firing, and c) a known attachment point of each ultrasound receiver (31), said prediction including:
determining an orientation of a virtual version of an unfired needle (30) relative to the planar ultrasound image derived from a length of a virtual positioning of a segment of the target biopsy needle (30) between the ultrasound receivers (31) relative to the planar ultrasound image as a function of the tracked positions of the ultrasound receivers (31); and
determining a tip extension of a virtual version of a fired needle (30) previously oriented relative to the planar ultrasound image derived from a length of a virtual positioning of a fired stylet of the target biopsy needle (30).

2. The ultrasound guide controller (44) of claim 1, wherein the trajectory prediction module (47) is configured to predict the biopsy trajectory as an in-plane biopsy trajectory responsive to the tracked positions of the at least two ultrasound receivers (31) indicating the at least two ultrasound receivers (31) being within the ultrasound plane.

3. The ultrasound guide controller (44) of claim 1, wherein the trajectory prediction module (47) is configured to predict the biopsy trajectory as an out-of-plane biopsy trajectory responsive to the tracked positions of the at least two ultrasound receivers (31) indicating at least one of the at least two ultrasound receivers (31) being outside the ultrasound plane.

4. A target biopsy system, comprising the ultrasound guide controller (44) of claim 1 and further comprising:
the ultrasound probe (20); and
the target biopsy needle (30) having the at least two ultrasound receivers (31) arranged thereon.

5. The target biopsy system of claim 4, wherein the target biopsy needle (30) includes a firing mechanism operable to project the target biopsy needle (30) along the predicted biopsy trajectory of the target biopsy needle (30) within the anatomical region.

6. The target biopsy system of claim 4, wherein the target biopsy needle (30) includes a coaxial introducer operable to introduce the target biopsy needle (30) into the anatomical region.

7. The target biopsy system of clam 4,
wherein a distal ultrasound receiver (31) of the at least two ultrasound receivers (31) is adjacent a tip of the target biopsy needle (30); and
wherein each additional ultrasound receiver (31) of the at least two ultrasound receiver (31) are spatially arranged on the target biopsy needle (30).

8. The target biopsy system of claim 4, wherein the ultrasound guide controller (44) is configured to predict the biopsy trajectory as an in-plane biopsy trajectory responsive to the sensing of the ultrasound plane indicating the at least two ultrasound receivers (31) being within the ultrasound plane.

9. The target biopsy system of claim 8, wherein the ultrasound guide controller (44) is configured to predict the biopsy trajectory as an out-of-plane biopsy trajectory responsive to the sensing of the ultrasound plane indicating at least one of the at least two ultrasound receivers (31) being outside the ultrasound plane.

10. The target biopsy system of claim 4, further comprising:
a monitor (41) operable in communication with the ultrasound guide controller (44) to display the planar ultrasound image; and
wherein the ultrasound guide controller (44) is operable to control a display of a biopsy trajectory overlay on a planar ultrasound image of the anatomical region displayed by the monitor (41), the biopsy trajectory overlay being derived from a prediction of the biopsy trajectory of the target biopsy needle (30) within the anatomical region relative to the ultrasound plane.

11. The target biopsy system of claim 10, wherein the ultrasound guide controller (44) is configured to control the display of the biopsy trajectory overlay as an in-plane biopsy trajectory responsive to the at least two ultrasound receivers (31) being within the ultrasound plane.

12. The target biopsy system of claim 10, wherein the ultrasound guide controller (44) is configured to control the display of the biopsy trajectory overlay as an out-of-plane biopsy trajectory responsive to at least one of the at least two ultrasound receivers (31) being outside the ultrasound plane.

13. The target biopsy system of claim 10, further comprising:
an interface platform (42) operable in communication with the ultrasound guide controller (44) to control the display of the planar ultrasound image by the monitor (41).

## Patentansprüche

1. Eine Ultraschall-Führungssteuerung (44), die ein Zielbiopsiesystem steuert, das Folgendes umfasst:
i) eine
Ultraschallsonde (20), die eine Ultraschallebene projiziert, die eine anatomischen Region schneidet, und
ii) eine Zielbiopsienadel (30) mit mindestens zwei Ultraschallempfängern (31), die auf bekannte Weise relativ zur Zielbiopsienadel (30) auf dieser angeordnet sind, wobei die einzelnen Ultraschallempfänger (31) die Ultraschallebene erfassen, wenn die Zielbiopsienadel (30) in die anatomische Region eingeführt wird, wobei die Ultraschall-Führungssteuerung (44) Folgendes umfasst:
ein Ultraschall-Bildgebungsmodul (45), das anhand der von der Ultraschallsonde (20) empfangenen Ultraschalldaten ein planares Ultraschallbild der anatomischen Region generieren, wenn die einer Ultraschallebene entsprechenden Ultraschalldaten die anatomische Region schneiden;
ein Empfängerverfolgungsmodul (46), das anhand der von den mindestens zwei Ultraschallempfängern (31) empfangenen Abtastdaten die Positionen der einzelnen Ultraschallempfänger (31) relativ zur Ultraschallebene nachverfolgen, wenn der Ultraschallebene entsprechende Daten erfasst werden, sobald die Zielbiopsienadel (30) in die anatomische Region eingeführt wurde; und
ein Wegvorhersagemodul (47), das mit dem Ultraschall-bildgebungsmodul (45) und dem Empfängerverfolgungsmodul (46) kommuniziert, um den Biopsieweg der Zielbiopsienadel (30) relativ zur Ultraschallebene vorhersagt, wenn die nachverfolgten Positionen der mindestens zwei Ultraschallempfänger (31) relativ zum planaren Ultraschallbild der anatomischen Region erfasst wurden; und das sich dadurch auszeichnet, dass die Vorhersage auf
a) einer bekannten Länge der Zielbiopsienadel (30) vor dem Einführen, b) einer bekannten Länge der Zielbiopsienadel (30) nach dem Einführen, und c) den bekannten Befestigungspunkten der einzelnen Ultraschallempfänger (31) beruht, wobei die Vorhersage folgende Schritte umfasst:
Ermitteln der Ausrichtung einer virtuellen Version einer nicht eingeführten Nadel (30) relativ zum planaren Ultraschallbild, die aus der Länge der virtuellen Positionierung eines Segments der Zielbiopsienadel (30) zwischen den Ultraschallempfängern (31) relativ zum planaren Ultraschallbild als eine Funktion der nachverfolgten Positionen der Ultraschallempfänger (31) abgeleitet wird; und
Ermitteln der Spitzenverlängerung der virtuellen Version einer eingeführten und zuvor relativ zum planaren Ultraschallbild ausgerichteten Nadel (30), die von der Länge einer virtuellen Positionierung eines eingeführten Mandrins der Zielbiopsienadel (30) abgeleitet wird.

2. Die Ultraschallführungssteuerung (44) gemäß Anspruch 1, wobei das Wegvorhersagemodul (47) den Biopsieweg als Biopsieweg in der Ebene vorhersagt, wenn die nachverfolgten Positionen der mindestens zwei Ultraschallempfänger (31) angeben, dass die mindestens zwei Ultraschallempfänger (31) innerhalb der Ultraschallebene liegen.

3. Die Ultraschallführungssteuerung (44) gemäß Anspruch 1, wobei das Wegvorhersagemodul (47) den Biopsieweg als Biopsieweg außerhalb der Ebene vorhersagt, wenn die nachverfolgten Positionen der mindestens zwei Ultraschallempfänger (31) angeben, dass mindestens einer der mindestens zwei Ultraschallempfänger (31) außerhalb der Ultraschallebene liegt.

4. Ein Zielbiopsiesystem, das die Ultraschallführungssteuerung (44) gemäß Anspruch 1 und zudem Folgendes umfasst:
die Ultraschallsonde (20); und
die Zielbiopsienadel (30), auf der die mindestens zwei Ultraschallempfänger (31) angeordnet sind.

5. Das Zielbiopsiesystem gemäß Anspruch 4, wobei die Zielbiopsienadel (30) einen Aktivierungsmechanismus umfasst, mit dem die Zielbiopsienadel (30) entlang des vorhergesagten Biopsiewegs der Zielbiopsienadel (30) in der anatomischen Region projiziert wird.

6. Das Zielbiopsiesystem gemäß Anspruch 4, wobei die Zielbiopsienadel (30) eine koaxiale Einführvorrichtung umfasst, mit der Zielbiopsienadel (30) in die anatomische Region eingeführt wird.

7. Das Zielbiopsiesystem gemäß Anspruch 4,
wobei ein distaler Ultraschallempfänger (31) der mindestens zwei Ultraschallempfänger (31) neben einer Spitze der Zielbiopsienadel (30) angebracht ist; und wobei alle weiteren Ultraschallempfänger (31) der mindestens zwei Ultraschallempfänger (31) räumlich auf der Zielbiopsienadel (30) verteilt sind.

8. Das Zielbiopsiesystem gemäß Anspruch 4, wobei die Ultraschallführungssteuerung (44) den Biopsieweg als einen Biopsieweg in der Ebene vorhersagt, wenn eine Ultraschallebene erfasst wurde, die angibt, dass die mindestens zwei Ultraschallempfänger (31) in der Ultraschallebene liegen.

9. Das Zielbiopsiesystem gemäß Anspruch 8, wobei die Ultraschallführungssteuerung (44) den Biopsieweg als einen Biopsieweg außerhalb der Ebene vorhersagt, wenn eine Ultraschallebene erfasst wurde, die angibt, dass mindestens einer der mindestens zwei Ultraschallempfänger (31) außerhalb der Ultraschallebene liegt.

10. Das Zielbiopsiesystem gemäß Anspruch 4, das zudem Folgendes umfasst:
einen Monitor (41), der mit der Ultraschallführungssteuerung (44) kommuniziert, um das planare Ultraschallbild anzuzeigen; und
wobei die Ultraschallführungssteuerung (44) die Anzeige einer Biopsiewegüberlagerung auf dem auf dem Monitor (41) angezeigten planaren Ultraschallbild der anatomischen Region steuert, und wobei die Biopsiewegüberlagerung von der Vorhersage des Biopsiewegs der Zielbiopsienadel (30) in der anatomischen Region relativ zur Ultraschallebene abgeleitet wird.

11. Das Zielbiopsiesystem gemäß Anspruch 10, wobei die Ultraschallführungssteuerung (44) die Anzeige der Biopsiewegüberlagerung als Biopsieweg in der Ebene steuert, wenn sich die mindestens zwei Ultraschallempfänger (31) in der Ultraschallebene befinden.

12. Das Zielbiopsiesystem gemäß Anspruch 10, wobei die Ultraschallführungssteuerung (44) die Anzeige der Biopsiewegüberlagerung als Biopsieweg außerhalb der Ebene steuert, wenn sich mindestens einer der mindestens zwei Ultraschallempfänger (31) außerhalb der Ultraschallebene befindet.

13. Das Zielbiopsiesystem gemäß Anspruch 10, das zudem Folgendes umfasst:
eine Schnittstellenplattform (42), die mit der Ultraschallführungssteuerung (44) kommuniziert, um die Anzeige des planaren Ultraschallbilds auf dem Monitor (41) zu steuern.

## Revendications

1. Dispositif de commande du guide à ultrasons (44) configuré pour commander un système de biopsie ciblée, lequel comprend i) une
sonde à ultrasons (20) utilisable pour projeter un plan ultrasonore coupant une zone anatomique, et ii) une aiguille de biopsie ciblée (30) comportant au moins deux récepteurs à ultrasons (31) disposés sur celle-ci dans un agencement connu par rapport à l'aiguille de biopsie ciblée (30) et dans lequel chaque récepteur à ultrasons (31) est utilisable pour détecter le plan ultrasonore lorsque l'aiguille de biopsie ciblée (30) est insérée dans la zone anatomique, ledit dispositif de commande du guide à ultrasons (44) comprenant :
un module d'imagerie par ultrasons (45), configuré en fonction des données ultrasonores reçues de la sonde à ultrasons (20), pour générer une image ultrasonore planaire d'une zone anatomique en réponse aux données ultrasonores représentatives d'un plan ultrasonore coupant la zone anatomique ;
un module de suivi du récepteur (46), configuré en fonction des données de détection reçues des au moins deux récepteurs à ultrasons (31), pour suivre une position de chaque récepteur à ultrasons (31) par rapport au plan ultrasonore en réponse aux données de détection représentatives d'une détection du plan ultrasonore lorsque l'aiguille de biopsie ciblée (30) est insérée dans la zone anatomique ; et
un module de prédiction de trajectoire (47) utilisable en communication avec le module d'imagerie par ultrasons (45) et le module de suivi du récepteur (46) pour prédire une trajectoire de biopsie de l'aiguille de biopsie ciblée (30) par rapport au plan ultrasonore en réponse aux positions suivies des au moins deux récepteurs à ultrasons (31) par rapport à l'image ultrasonore planaire de la zone anatomique ;
**caractérisé par**
ladite prédiction étant basée sur
a) une longueur connue de l'aiguille de biopsie ciblée (30) avant déploiement, b) une longueur connue de l'aiguille de biopsie ciblée (30) après déploiement, et c) un point de fixation connu de chaque récepteur à ultrasons (31), ladite prédiction comprenant :
la détermination d'une orientation d'une version virtuelle d'une aiguille non déployée (30) par rapport à l'image ultrasonore planaire dérivée d'une longueur d'un positionnement virtuel d'un segment de l'aiguille de biopsie ciblée (30) entre les récepteurs à ultrasons (31) par rapport à l'image ultrasonore planaire en fonction des positions suivies des récepteurs à ultrasons (31) ; et
la détermination d'une extension de pointe d'une version virtuelle d'une aiguille déployée (30) préalablement orientée par rapport à l'image ultrasonore planaire dérivée d'une longueur d'un positionnement virtuel d'un stylet déployé de l'aiguille de biopsie ciblée (30).

2. Dispositif de commande du guide à ultrasons (44) selon la revendication 1, dans lequel le module de prédiction de trajectoire (47) est configuré pour prédire la trajectoire de biopsie en tant que trajectoire de biopsie dans le plan en réponse aux positions suivies des au moins deux récepteurs à ultrasons (31) indiquant les au moins deux récepteurs à ultrasons (31) se trouvant dans le plan à ultrasons.

3. Dispositif de commande du guide à ultrasons (44) selon la revendication 1, dans lequel le module de prédiction de trajectoire (47) est configuré pour prédire la trajectoire de biopsie en tant que trajectoire de biopsie hors plan en réponse aux positions suivies des au moins deux récepteurs à ultrasons (31) indiquant au moins l'un des au moins deux récepteurs à ultrasons (31) étant à l'extérieur du plan ultrasonore.

4. Système de biopsie ciblée, comprenant le dispositif de commande du guide à ultrasons (44) selon la revendication 1 et comprenant en outre :
la sonde à ultrasons (20) ; et
l'aiguille de biopsie ciblée (30) comportant les au moins deux récepteurs à ultrasons (31) disposés sur celle-ci.

5. Système de biopsie ciblée selon la revendication 4, dans lequel l'aiguille de biopsie ciblée (30) comprend un mécanisme de déclenchement utilisable pour déployer l'aiguille de biopsie ciblée (30) le long de la trajectoire de biopsie prédite de l'aiguille de biopsie ciblée (30) dans la zone anatomique.

6. Système de biopsie ciblée selon la revendication 4, dans lequel l'aiguille de biopsie ciblée (30) comprend un introducteur coaxial utilisable pour introduire l'aiguille de biopsie ciblée (30) dans la zone anatomique.

7. Système de biopsie ciblée selon la revendication 4,
dans lequel un récepteur à ultrasons (31) distal des au moins deux récepteurs à ultrasons (31) est adjacent à une pointe de l'aiguille de biopsie ciblée (30) ; et
dans lequel chaque récepteur à ultrasons (31) supplémentaire des au moins deux récepteurs à ultrasons (31) est disposé spatialement sur l'aiguille de biopsie ciblée (30).

8. Système de biopsie ciblée selon la revendication 4, dans lequel le dispositif de commande du guide à ultrasons (44) est configuré pour prédire la trajectoire de biopsie en tant que trajectoire de biopsie dans le plan en réponse à la détection du plan ultrasonore indiquant que les au moins deux récepteurs à ultrasons (31) se trouvent dans le plan ultrasonore.

9. Système de biopsie ciblée selon la revendication 8, dans lequel le dispositif de commande du guide à ultrasons (44) est configuré pour prédire la trajectoire de biopsie en tant que trajectoire de biopsie hors plan en réponse à la détection du plan ultrasonore indiquant l'au moins un des au moins deux récepteurs à ultrasons (31) se trouvant hors du plan ultrasonore.

10. Système de biopsie ciblée selon la revendication 4, comprenant en outre :
un moniteur (41) utilisable en communication avec le dispositif de commande du guide à ultrasons (44) pour afficher l'image ultrasonore planaire ; et
dans lequel le dispositif de commande du guide à ultrasons (44) est utilisable pour commander un affichage d'une superposition de trajectoire de biopsie sur une image ultrasonore planaire de la zone anatomique affichée par le moniteur (41), la superposition de trajectoire de biopsie étant dérivée d'une prédiction de la trajectoire de biopsie de l'aiguille de biopsie ciblée (30) dans la zone anatomique par rapport au plan ultrasonore.

11. Système de biopsie ciblée selon la revendication 10, dans lequel le dispositif de commande du guide à ultrasons (44) est configuré pour commander l'affichage de la superposition de trajectoire de biopsie en tant que trajectoire de biopsie dans le plan en réponse aux au moins deux récepteurs à ultrasons (31) se trouvant dans le plan ultrasonore.

12. Système de biopsie ciblée selon la revendication 10, dans lequel le dispositif de commande du guide à ultrasons (44) est configuré pour commander l'affichage de la superposition de trajectoire de biopsie en tant que trajectoire de biopsie hors plan en réponse à l' au moins un des au moins deux récepteurs à ultrasons (31) se trouvant hors du plan ultrasonore.

13. Système de biopsie ciblée selon la revendication 10, comprenant en outre :
une plate-forme interface (42) utilisable en communication avec le dispositif de commande du guide à ultrasons (44) pour commander l'affichage de l'image ultrasonore planaire par le moniteur (41).
